Europäisches Patentamt

European Patent Office

Office européen des brevets

(19)

(11) Publication number: **0 098 534**
**B1**

(12) # EUROPEAN PATENT SPECIFICATION

(45) Date of publication of patent specification: **10.09.86**

(51) Int. Cl.⁴: **G 01 N 33/48, B 01 D 25/04**

(21) Application number: **83106390.4**

(22) Date of filing: **30.06.83**

(54) **Filter apparatus.**

(30) Priority: **01.07.82 US 394225**

(43) Date of publication of application:
**18.01.84 Bulletin 84/03**

(45) Publication of the grant of the patent:
**10.09.86 Bulletin 86/37**

(84) Designated Contracting States:
**CH DE FR GB LI SE**

(56) References cited:
GB-A-2 000 694
US-A- 30 562
US-A- 768 605
US-A-3 111 489
US-A-3 158 532
US-A-3 238 056
US-A-3 523 408
US-A-3 540 856
US-A-3 540 857
US-A-3 540 858
US-A-3 649 464
US-A-4 154 795
US-A-4 276 048
US-A-4 304 865
US-A-4 459 139

(73) Proprietor: **MILLIPORE CORPORATION**
**80 Ashby Road**
**Bedford Massachusetts 01730 (US)**

(72) Inventor: **Champion, Helena Margaret**
**228 Puritan Rd.**
**Swampscott MA, 01907 (US)**
Inventor: **Pierog, Joseph John**
**3 Mildred St.**
**Salem N.H. 03079 (US)**
Inventor: **Peters, Joseph Edward**
**148 Fifty Acre Way**
**Carlisle MA 01741 (US)**

(74) Representative: **Patentanwälte Zellentin**
**Zweibrückenstrasse 15**
**D-8000 München 2 (DE)**

Courier Press, Leamington Spa, England.

EP 0 098 534 B1

## Description

The invention relates to laboratory apparatus useful in the assay of biological and biochemical reactants and is particularly concerned with multi-well filtration devices able to retain fluids for substantial periods of time before filtration is performed.

Test plates for in vitro analysis which contain a multiplicity of individual wells or reaction chambers are commonly known laboratory tools. Such devices have been employed for a broad variety of purposes and assays as are exemplified by U.S. Patent Nos. 3,649,464; 4,304,865; 4,276,048; 4,154,795; and Re 30,562. Microporous membrane filters and filtration devices containing such microporous membranes have become especially useful with many of the recently developed cell and tissue culture techniques and assays — particularly those in the fields of virology and immunology.

Typically, a 96-well filtration plate is used to conduct multiple assays simultaneously some of which last several hours before filtration is actually performed. With such filtration plates, especially those containing microporous membranes, there is a well recognized and recurrent problem in that fluids in the wells tend to pass through the membrane by capillary action and gravity flow thereby causing a loss of contents from within the reaction well before the desired stage in the experimental design. Prevention of fluid loss by capillary action and gravity flow becomes especially important when living cells or tissues are being maintained or grown within the reaction wells. Under these circumstances, favorable media conditions for the cells or tissues must be maintained for hours or even days and any loss of fluid from the wells, however small, will affect the condition of the cells and influence the results of the assay. Prevention of fluid loss through the membrane in this manner is also vitally important when the assay utilizes very small sample volumes as reactants, such test samples often being less than 100 microliters in volume. The pendant drop that invariably forms on the underside of the microporous membrane due to such capillary action and gravity flow is typically about 50 microliters in volume and it is apparent that a fluid loss of such proportions must drastically affect the assay.

Nevertheless, insofar as is presently known, no filtration apparatus has been able to prevent this loss of fluid from the reaction well, particularly under small sample volume assay conditions.

It is the object of the present invention to provide a filtration apparatus for the assay of microliter quantities of biological and biochemical reactants, which apparatus is able to retain liquid for extended periods of time without a loss of fluid, and allows filtration of said liquid at any moment desired.

According to the present invention, this object is achieved by a filtration apparatus able to retain liquid for extended periods of time comprising: a plate having at least one aperture open at each end; filtration means disposed over one end of said aperture in said plate such that a well having a discrete filtering area is formed; and a hydrophobic fabric attached to said filtration means adjacent to said filtering area.

The hydrophobic fabric prevents a loss of fluid by capillary action and gravity flow from within the well in the absence of an applied differential pressure. Additionally provided are fluid collection means and a guiding projection which directs such fluid as passes through the filtration means to a predetermined location within the fluid collection means.

The present invention may be best understood when taken in conjunction with the accompanying drawing, in which:

Fig. 1 is an expanded view of a vacuum assembly useful with the invention;

Fig. 2 is an overhead view of a filtration apparatus comprising one embodiment of the present invention;

Fig. 3 is a cross-sectional view of the preferred filtration apparatus comprising the present invention;

Fig. 4 in one embodiment of fluid collections means useful with the preferred embodiment illustrated in Fig. 3; and

Fig. 5 is another preferred embodiment of the invention illustrated in Fig. 3.

The invention is an improvement in filtration apparatus having at least one reaction well which typically contains a microporous membrane for the separation and retention of matter from fluids. Attached adjacent to the microporous membrane is a porous hydrophobic fabric which is situated either above or preferably below the filtering microporous membrane. This hydrophobic fabric prevents fluid loss by capillary action and gravity flow through the membrane in the absence of a vacuum force but will still allow diffusion of gases into or out the interior of each well on the plate.

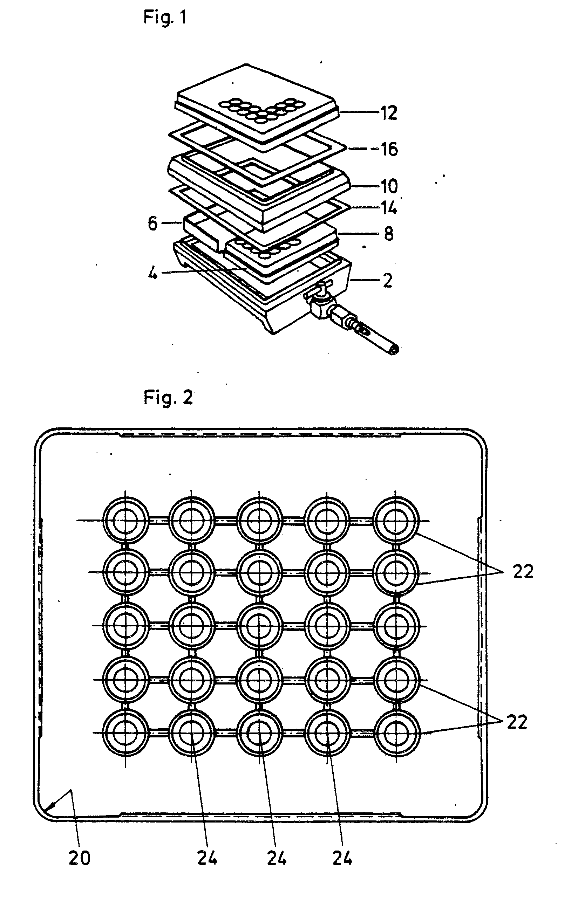

Embodiments of the invention are most useful with the vacuum assembly shown in Fig. 1 which is capable of simultaneously processing 96 individual test samples of up to 440 microliters (μl) each. The vacuum assembly comprises a base 2 which acts as a vacuum chamber and contains a hose barb for connection to a regulated external vacuum source. Housed within the base 2 are fluid collection means 4 which include a collection tray 6 and/or a receiving plate 8 having up to 96 individual chambers for the collection of filtrate. A filter support 10 holding a 96-well filtration plate 12 lies above the fluid collection means 4 separated by gaskets 14 and 16 which form an airtight seal in the presence of a vacuum force.

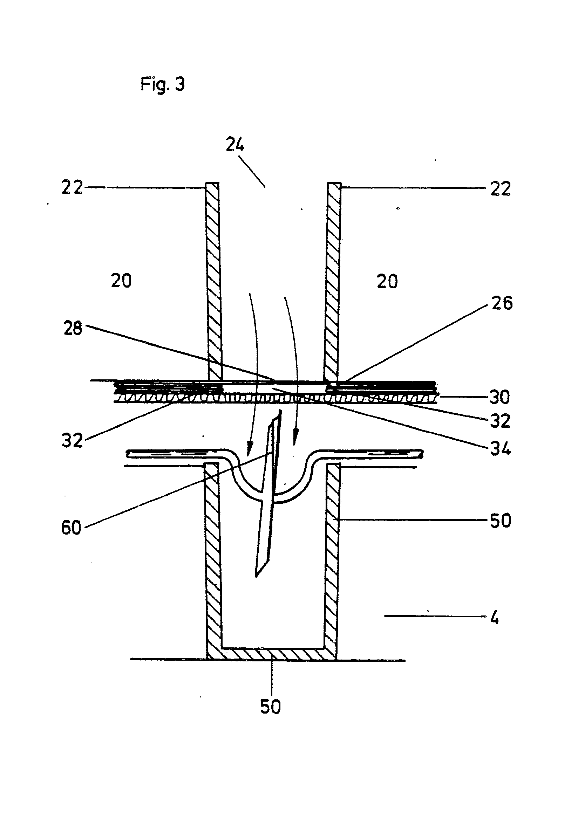

Detailed views of the filtration plate utilizing the preferred embodiment of the present invention are shown in Figs. 2 and 3. It will be appreciated that the number of wells found in the filtration plate are simply a matter of convenience for the investigator. The plate 20 may contain as few as one well or as many wells as are functionally permissible given the actual dimensions of the plate.

The filtration plate may be formed of any resilient and nonreactive material commonly available, the composition of choice being a matter of convenience or economics only. Each well 22 comprises an aperture 24 through the entire depth of the plate, the thickness of the plate determining the volume of fluid to be retained within the well. The diameter of the aperture will vary to meet the user's needs but typically will range from 3 to 25 millimeters in diameter. The filtration means 26, typically a microporous membrane filter, is disposed across and sealed about the aperture 24 in the plate 20 such that the area across each well will serve as a filtering area 28. Methods of bonding the microporous membrane to the plate and sealing it about the perimeter of the aperture 24 are well known in the art and need not be described in detail here. The composition and flow characteristics of the filtration means 26 forming the filtering area 28 across each aperture 24 is also a matter of choice. Typically nitrocellulose membranes cellulose acetate, polycarbonate and polyvinylidene fluoride microporous membranes are selected because of their proven characteristics in aqueous solutions and tissue culture media. The porosity of the membrane will be selected with a view to the chosen application. Although 0.025 to 10.0 micrometer prosity membranes of 150 micrometers thickness are favored, the filtration means 26 are not limited to microporous membranes as such. Rather, ultrafiltration media can be utilized in lieu of microporous membrane. By the term ultrafiltration media is meant a material capable of retaining a molecule in solution. Such ultrafiltration media are useful for retaining molecules as small as about 100 daltons and generally molecules as large as about two million daltons. Examples of such ultrafiltration media are well known in the art and include polysulfone and other polymeric materials available from Millipore Corporation under the registered trademark, PELLICON®. Similarly, macrofiltration media such as glass fiber for retention of gross particles may be used. It will be appreciated by those ordinarily skilled in the art that the individual filtering areas 28 bounded by each well 22 can be removed via a filter punch after filtration for further processing if necessary.

As can be seen in Fig. 3, a hydrophobic fabric 30 is disposed across and bonded adjacent to the filtering areas 28 of the well 22. Preferably, the hydrophobic fabric is bonded to the filtration means abutting the well perimeter 32 such that a minute space 34 is created and maintained between the fabric 30 and the filtering area 28. The fabric 30 many be heat bondable or utilize and adhesive for attachment to the filtration means 26. In addition, the fabric 30 may be formed of woven or a nonwoven materials and be composed any of hydrophobic polyester, polyolefin, polytetrafluoroethylene or other polymer — many suitable varieties being commercially available.

It is preferred that attachment of the filtration means 26 and the hydrophobic fabric 30 to the plate 20 be performed as separate steps to insure their proper positioning and the formation of the minute space 34. Nevertheless, it is possible to attach both the filtration means and the hydrophobic fabric simultaneously, particularly if a heat bondable hydrophobic material is used as the fabric layer.

Affixation of a porous hydrophobic fabric in this manner permits the use of small sample volumes, often less than 100 microliter (hereinafter μl), to be used as reactants. Without the fabric layer, a drop of fluid approximately 50 μl in volume will collect below the filtration means as a pendant drop and become lost. With the hydrophobic fabric in place, the pendant drop that forms below the filtering area 28 as a result of capillary action and gravity flow will be substantially retained within minute space 34 and the tendency for liquid to pass through the filtering area is substantially reduced or entirely eliminated. As a result, assays during which the well contents require a fluid media incubation phase or a bathing of the reactants in fluid can be performed without errors or inconvenience.

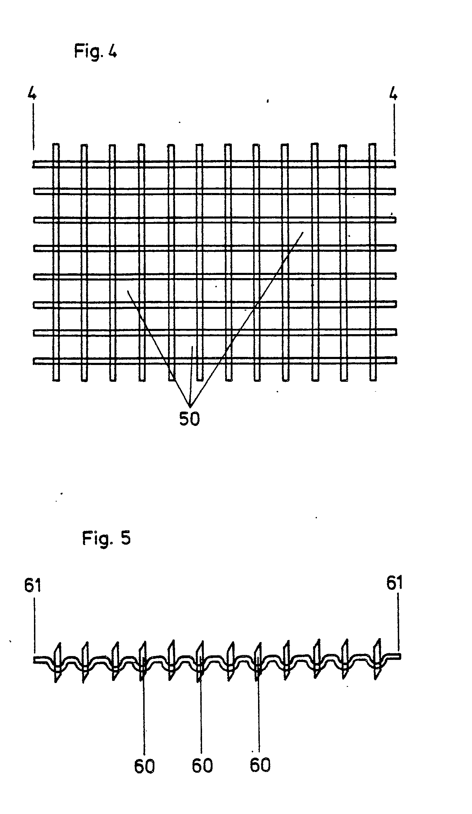

Another aspect of the present invention is the pendant drop release fixture illustrated in Figs. 3 and 5. This fixture is intended to be used with the multichambered fluid collection means shown in Figs. 1 and 4 which is designed to receive filtrate from the interior of the well aligned directly above it via a plurality of individual receiving chambers 50. In this manner, the filtrate from each well will be retained separately. This compartmentalization feature alone, however, may not correct for the problem of comingling of filtrates deriving from different wells as the fluid is pulled through the hydrophobic fabric by an applied differential pressure. Similary, in those situations where the hydrophobic fabric is not present or is not necessary for the purposes of the assay, pendant drops will form and routinely collect on the underside of each filtering area. In small volume assays, the worker cannot afford to lose the 50 μl hanging as a drop from the membrane. Even in larger volume assays, an accidental movement or subsequent manipulations of the filter plate will dislodge the pendant drop and cause it to fall into the wrong receiving chamber causing cross-contamination of filtrates and erroneous test results.

Both these kinds of problems are corrected by placement of a pendant drop release fixture — in the form of a guiding projection 60 — between the filtering area 28 and the fluid collection means 4 beneath the plate 20. The preferred embodiment of this guiding projection 60 appears in Figs. 3 and 5 as a series of spikes 60 molded in a pattern corresponding to the individual filtering area 28 in the plate 20. Each spike 60 serves a dual function: first, as a surface upon which the small volumes of fluid which would otherwise be lost as a pendant drop are collected and removed from the filtering area 28; second, as a guide by which the fluids forming a pendant drop are directed to the appropriate chamber 50 in the fluid collection means 4. The projections 60 can be injection molded or a die cut assembly. Any molding polymer material such as nylon, polystyrene, polycarbonate and polyethylene may be used for making the guiding projections; however, a hydrophilic material is preferred because it promotes interception and guidance of the pendant drop.

It is expected that the hydrophobic fabric and the fluid guiding projection will be used in tandem in the majority of assays. Nevertheless, where retention of fluid within the well is not necessary, the pendant drop release fixture may be used alone to advantage.

## Claims

1. A filtration apparatus able to retain liquid for extended periods of time comprising:
   a plate having at least one aperture open at each end;
   filtration means disposed over one end of said aperture in said plate such that a well having a discrete filtering area is formed; and
   a hydrophobic fabric attached to said filtration means adjacent to said filtering area.

2. A filtration apparatus as recited in claim 1, which further comprises a projection aligned beneath said filtration means and said hydrophobic fabric such that fluid passing through said filtering area is directed to a predetermined location.

3. The filtration apparatus as recited in claim 1 or 2, wherein said filtration means includes a microporous membrane.

4. The filtration apparatus as recited in claim 3 wherein said filtration means includes a microporous membrane having a pore size of at least 25 nm.

5. The filtration apparatus as recited in claim 1 or 2 wherein said filtration means retain molecules from one hundred daltons to two million daltons.

6. The filtration apparatus as recited in claim 1 or 2 wherein said hydrophobic fabric is selected from woven or nonwoven polymers.

7. The filtration apparatus as recited in claim 1 or 2 wherein said hydrophobic fabric is selected from polyesters, polyolefins and polytetrafluoroethylene.

8. The filtration apparatus as recited in claim 1 or 2 wherein said hydrophobic fabric is heat bondable.

9. The filtration apparatus as recited in claim 1 or 2 wherein said hydrophobic fabric is attached with adhesive.

10. The filtration apparatus as recited in claim 1 or 2 further comprising fluid collection means disposed beneath said filtration means.

11. The filtration apparatus as recited in claim 10 wherein said fluid collection means includes a plurality of individual receiving chambers.

12. The filtration apparatus as recited in claim 2 wherein said projection is disposed upon fluid collection means.

13. The filtration apparatus as recited in any of the preceding claims further comprising means for applying a differential pressure on liquid placed in said well to effect passage of said liquid through said filtration means and said hydrophobic fabric.

14. The filtration apparatus as recited in any of the preceding claims, wherein the hydrophobic fabric is bonded to the filtration means such that a minute space is maintained between the fabric and the filtering area.

## Revendications

1. Appareil de filtration capable de retenir un liquide pendant des périodes de temps prolongées, comportant:
   une plaque présentant au moins une ouverture ouverte à chaque extrémité; un moyen de filtration disposé au-dessus de l'une des extrémités de ladite ouverture dans ladite plaque de façon qu'il se forme un puits présentant une zone filtrante distincte; et un tissu hydrophobe fixé au dit moyen de filtration près de ladite zone filtrante.

2. Appareil de filtration comme exposé dans la revendication 1, comportant de plus une saillie alignée sous ledit moyen de filtration et sous ledit tissu hydrophobe de façon que le fluide passant à travers ladite zone filtrante soit dirigé vers un emplacement prédéterminé.

3. Appareil de filtration comme exposé dans la revendication 1 ou 2, où ledit moyen de filtration inclut une membrane microporeuse.

4. Appareil de filtration comme exposé dans la revendication 3, où ledit moyen de filtration inclut une membrane microporeuse d'une dimension de pore d'au moins 25 nm.

5. Appareil de filtration comme exposé dans la revendication 2 où ledit moyen de filtration retient des molécules allant de cent daltons à deux millions de daltons (1,68. $10^{-22}$ g à 3,36. $10^{-18}$ g).

6. Appareil de filtration comme exposé dans la revendication 1 ou 2 où ledit tissu hydrophobe est choisi parmi des polymères tissés ou non tissés.

7. Appareil de filtration comme exposé dans la revendication 1 ou 2, où ledit tissu hydrophobe est choisi parmi des polyesters, des polyoléfines et le polytétrafluoroéthylène.

8. Appareil de filtration comme exposé dans la revendication 1 ou la revendication 2, où ledit tissu hydrophobe est soudable à chaud.

9. Appareil de filtration comme exposé dans la revendication 1 ou 2, où ledit tissu hydrophobe est fixé avec de la colle.

10. Appareil de filtration comme exposé dans la revendication 1 ou 2, comportant en outre un moyen de collecte du fluide disposé sous ledit moyen de filtration.

11. Appareil de filtration comme exposé dans la revendication 10 où ledit moyen de collecte du fluide inclut un certain nombre de chambres individuelles de réception.

12. Appareil de filtration comme exposé dans la revendication 2 où ladite saillie est disposée sur le moyen de collecte du fluide.

13. Appareil de filtration comme exposé dans l'une quelconque des revendications précédentes, comportant en outre un moyen d'appliquer une pression différentielle sur le liquide placé dans ledit puits pour faire passer ledit liquide à travers ledit moyen de filtration et à travers ledit tissu hydrophobe.

14. Appareil de filtration comme exposé dans l'une quelconque des revendications précédentes, où le tissu hydrophobe est relié au moyen de filtration de façon telle qu'un très petit espace soit maintenu entre le tissu et la zone filtrante.

**Patentansprüche**

1. Ein Filtergerät, das in der Lage ist, Flüssigkeit über ausgedehnte Zeiträume zurückzuhalten, mit:

einer Platte mit wenigstens einer an beiden Enden offenen Öffnung;

Filtermitteln, die über einem Ende der genannten Öffnung in der genannten Platte angeordnet sind, so daß eine Vertiefung mit einem abgegrenzten Filterbereich gebildet wird; einem hydrophoben Gewebe, das angrenzend an den genannten Filterbereich an die genannten Filtermittel befestigt ist.

2. Filtergerät nach Anspruch 1, das außerdem ein vorspringendes Teil umfaßt, das so unterhalb der genannten Filtermittel und des genannten hydrophoben Gewebes ausgerichtet ist, daß ein Fluid, das durch den genannten Filterbereich hindurchtritt, zu einem vorgegebenen Ort geleitet wird.

3. Filtergerät nach Anspruch 1 oder 2, bei dem die genannten Filtermittel eine mikroporöse Membran umfassen.

4. Filtergerät nach Anspruch 3, bei dem die genannten Filtermittel eine mikroporöse Membran mit einer Porengröße von wenigstens 25 nm umfassen.

5. Filtergerät nach Anspruch 1 oder 2, bei dem die genannten Filtermittel Moleküle von 100 Dalton bis 2 Millionen Dalton zurückhalten.

6. Filtergeräte nach Anspruch 1 oder 2, bei dem das genannte hydrophobe Gewebe ausgewählt ist aus gewebten und nichtgewebten Polymeren.

7. Filtergerät nach Anspruch 1 oder 2, bei dem das genannte hydrophobe Gewebe ausgewählt ist aus Polyestern, Polyolefinen und Polytetrafluoräthylen.

8. Filtergerät nach Anspruch 1 oder 2, bei dem das genannte hydrophobe Gewebe heißsiegelbar ist.

9. Filtergerät nach Anspruch 1 oder 2, bei dem das genannte hydrophobe Gewebe mit einem Klebstoff befestigt ist.

10. Filtergerät nach Anspruch 1 oder 2, das außerdem Mittel zum Sammeln des Fluids umfasst, die unterhalb der genannten Filtermittel angeordnet sind.

11. Filtergerät nach Anspruch 10, bei dem die genannten Mittel zum Sammeln eines Fluids eine Vielzahl von individuellen Aufnahmekammern umfassen.

12. Filtergerät nach Anspruch 2, bei dem das genannte vorspringende Teil über einem Mittel zum Sammeln des Fluids angeordnet ist.

13. Filtergerät nach einem der vorausgehenden Ansprüche, das außerdem Mittel zum Anlegen eines Druckunterschieds an eine Flüssigkeit, die sich in der genannten Vertiefung befindet, um den Durchtritt der genannten Flüssigkeit durch die genannten Filtermittel und das genannte hydrophobe Gewebe zu bewirken, umfaßt.

14. Filtergerät nach einem der vorausgehenden Ansprüche, bei dem das hydrophobe Gewebe so an die Filtermittel gebunden ist, daß zwischen dem Gewebe und dem Filterbereich ein kleiner Raum frei bleibt.

Fig. 1

Fig. 2

Fig. 3

Fig. 4

50

Fig. 5

61                                                    61

60      60      60